# EUROPÄISCHE PATENTSCHRIFT

(11) **EP 3 217 576 B1**
(45) Veröffentlichungstag und Bekanntmachung des Hinweises auf die Patenterteilung: **14.10.2020**
(21) Anmeldenummer: 17153992.7
(22) Anmeldetag: 31.01.2017
(51) Int. Cl.: H04B 11/00, G08C 23/02, H01L 41/00, H04R 17/10

(54) **IMPLANTAT UND VERFAHREN ZUM BETREIBEN DESSELBEN**
IMPLANT AND METHOD FOR OPERATING THE SAME
IMPLANT ET MISE EN OEUVRE ASSOCIEE

(30) Priorität: 07.03.2016 DE 102016104097
(43) Veröffentlichungstag der Anmeldung: 13.09.2017
(73) Patentinhaber: BIOTRONIK SE & Co. KG, 12359 Berlin (DE)
(72) Erfinder: Moß, Christian, 10589 Berlin (DE)
(74) Vertreter: Galander, Marcus

(56) Entgegenhaltungen:
- DE-A1-102012 112 237
- US-A1- 2002 045 921
- US-A1- 2011 218 594
- US-A1- 2014 336 474
- US-B1- 7 522 962

## Beschreibung

Die Erfindung betrifft ein medizinisches Implantat sowie ein Verfahren zum Betreiben desselben.

Medizinische Endoprothesen oder Implantate für die unterschiedlichsten Anwendungen sind in großer Vielfalt aus dem Stand der Technik bekannt. Implantate werden unterteilt in passive und aktive Implantate. Passive Implantate erfüllen eher mechanische Aufgaben im Körper, in den sie eingesetzt sind. Passive Implantate sind beispielsweise ein Stent, ein Herzklappenersatz, ein Brustimplantat, Prothesen von Körpergliedern oder optische Linsen. Demgegenüber beinhalten aktive Implantate eine Elektronik und sind in der Lage, bestimmte Körperzustände zu detektieren oder eine Behandlung vorzunehmen. Beispiele für aktive Implantate sind Herzschrittmacher, Neurostimulatoren, Medikamentenpumpen oder Defibrillatoren. Weitere Anwendungen aktiver Implantate umfassen auch Sensoren für Körperfunktionen, beispielsweise Drucksensoren wie sie in der Druckschrift US 2002/0045921 A1 beschrieben sind. Die vorliegende Erfindung beschäftigt sich mit aktiven Implantaten nach der obigen Unterscheidung.

Bei der Verwendung von aktiven Implantaten ist es erforderlich, Daten, die die Steuerung des Implantats betreffen oder die von dem Implantat erzeugt wurden, z. B. Messdaten eines in das Implantat integrierten Sensors, mit einer bezüglich des Implantats externen Einheit auszutauschen. Bei einer solchen, bezüglich des Implantats externen Einheit kann es sich beispielsweise um eine körperexterne Einheit wie z. B. um ein Programmiergerät oder um ein weiteres Implantat handeln. Zur Vereinfachung der Darstellung wird im Folgenden eine vorgenannte körperexterne Einheit oder ein weiteres Implantat als weiteres Gerät bezeichnet. Es ist insbesondere gewünscht, dass ein Datenaustausch mit dem weiteren Gerät drahtlos erfolgt. Zudem sollen die Implantate möglichst klein sein, um den Körper aufgrund ihres Raumbedarfs möglichst wenig zu belasten.

In bekannten Implantaten werden vorrangig galvanische Kommunikation/ impedanzmodulationsbasierte Kommunikation, induktive Nahfeldtelemetrie- oder Funksysteme zur Datenübertragung zwischen dem Implantat und einem körperextemen Gerät zur Datenerfassung und/oder Datenverarbeitung eingesetzt. Die bekannten Systeme haben jedoch eine Reihe von Nachteilen. Im Fall der galvanischen Kommunikation/ impedanzmodulationsbasierten Kommunikation sind Gehäusedurchführungen bzw. Elektroden an der Gehäuseaußenseite erforderlich. Zudem müssen auf der Seite des körperexternen Gerätes Elektroden auf der Körperoberfläche aufgeklebt werden, was bei einer häufigen Verwendung der Schnittstelle nur begrenzt praxistauglich ist. Die induktive Nahfeldtelemetrie besitzt einen stark eingeschränkten Übertragungsradius, insbesondere bei der Miniaturisierung von Implantaten und der damit verbundenen Verwendung von kleinen Spulen. In Bezug auf eine Funkübertragung von Daten besteht der bekannte Nachteil, dass die Antenne eine gewisse Größe aufweisen muss, was eine weitere Miniaturisierung verhindert. Zudem ist es nicht möglich, die Antenne in ein Gehäuse aus leitfähigem Material, wie beispielsweise Titan, zu platzieren, sodass die Integration einer Antenne in ein Implantat mit größerem Aufwand verbunden ist. Ein weiterer bekannter Nachteil bei der Funkübertragung von Daten besteht darin, dass der Energiebedarf für die Datenübertragung in Abhängigkeit von der Implantationstiefe des Implantats im Körper steigt. Dies führt zwangsläufig zum Einsatz größerer Energiequellen, was ebenfalls einer weiteren Miniaturisierung entgegensteht.

Auch Ultraschall wurde bereits zum Austausch von Daten mit einem Implantat verwendet. Die bisher vorgeschlagenen Systeme sind jedoch technologisch aufwändig und sehr platzintensiv. Beispiele für die Verwendung von Ultraschall zur Datenübertragung sind in den Druckschriften "Deeply implanted medical device based on a novel ultrasonic telemetry technology", These No. 5730 (2013) an der Ecole Polytechnique Federale de Lausanne von M. Peisino, US 5,861,018, US 2010/0249882 A1 oder US 2002/0045921 A1 beschrieben. Die Implantate nach dem Stand der Technik weisen ein Piezoelement auf, mit dem von einer externen Sendeeinheit ausgesandte Ultraschallsignale empfangen und in elektrische Signale gewandelt werden können.

In der Druckschrift US 6,140,740 wird ein passives Verfahren der Ultraschall-Kommunikation beschrieben, bei dem eine von einem externen Sender stammende Schallwelle in der Reflexion moduliert wird. Das für das Verfahren notwendige Patientengerät und die Produktion des Schallwandlers sind jedoch sehr aufwändig.

Das Dokument US 2011/0218594 A1 offenbart einen externen Ultraschallwandler für eine bidirektionale Kommunikation zwischen einem Implantat und dem Ultraschallwandler.

Das Dokument DE 10 2012 112 237 A1 offenbart ein elektroakustisches Bandpassfilter mit geglätteter Einfügedämpfung.

Das Dokument US 2014/0336474 A1 offenbart ein System zur Kommunikation zwischen einem Implantat und einem externen Gerät.

Folglich besteht die Aufgabe der vorliegenden Erfindung darin, ein Implantat mit der Möglichkeit der drahtlosen Datenübertragung zu schaffen, das einfach aufgebaut ist und eine weitere Miniaturisierung erlaubt.

Die obige Aufgabenstellung wird gelöst durch ein Implantat mit den in Anspruch 1 angegebenen Merkmalen.

Das erfindungsgemäße Implantat hat den Vorteil, dass für das Empfangen der ersten Ultraschallsignale und das Senden der zweiten Ultraschallsignale ein und dasselbe Piezoelement verwendet wird und dieses damit als Transceiver betrieben wird. Hierdurch ist nur ein geringer technologischer Aufwand für das Senden der ersten Ultraschallsignale und das Empfangen der zweiten Ultraschallsignale erforderlich. Zudem wird das Piezoelement beim Senden und Empfangen der Ultraschallsignale im Resonanzmodus betrieben, so dass eine effektive Energiewandlung erfolgen kann. Für einen Betrieb im Resonanzmodus können als Resonanzfrequenz beim Senden und beim Empfangen sowohl die Serienresonanzfrequenz als auch die Parallelresonanzfrequenz genutzt werden. Aus Gründen der Energieeffizienz ist es jedoch vorteilhaft, beim Senden und beim Empfangen der Ultraschallsignale, die Serienresonanzfrequenz zu nutzen.

Das Piezoelement erzeugt durch das Umwandeln der empfangenen ersten Ultraschallsignale ausreichend elektrische Energie, um bei der zweiten Resonanzfrequenz zweite Ultraschallsignale zu erzeugen und zu versenden. Erfindungsgemäß wurde demnach für das aktive Implantat ein passives Kommunikationsverfahren realisiert, bei dem die von den ersten Ultraschallsignalen umgewandelte elektrische Energie genutzt wird, um damit das Piezoelement bei der zweiten Resonanzfrequenz zum Schwingen anzuregen, wodurch das Piezoelement Ultraschall abgibt, der mittels eines weiteren Geräts empfangen werden kann. Das erfindungsgemäße Implantat kann in einem Fall ohne zusätzliche Energiequelle betrieben werden. In diesem Fall ist eine erhebliche Raumersparnis und entsprechende Miniaturisierung des Implantats möglich. In einem anderen Fall kann das Implantat mit einer Energiequelle betrieben werden. Dabei kann mit dem erfindungsgemäßen Implantat eine Energieeinsparung insbesondere bei häufig kommunizierenden Anwendungen erzielt werden, so dass eine deutliche Verlängerung der Lebensdauer des Implantats und/oder Reduzierung der Baugröße der Energiequelle des Implantats und damit eine Reduzierung der Baugröße des Implantats erreicht werden kann.

Im Zusammenhang mit der Übertragung von Ultraschallsignalen ist auch die Verwendung eines metallischen Gehäuses für das Implantat unproblematisch.

Gegenüber nicht-ultraschallbasierten Datenübertragungsverfahren ergibt sich somit durch die erfindungsgemäße Lösung ein deutlicher Vorteil im Hinblick auf eine bessere Integrierbarkeit und Miniaturisierung. Zudem lassen sich Schallwandler in Form von Piezoelementen leicht in hermetisch abgeschlossene und miniaturisierte Gehäuse integrieren.

Weiter ist vorteilhaft, dass mit dem erfindungsgemäßen Implantat auch eine Kommunikation zwischen zwei oder mehr Implantaten in einem Netzwerk realisiert werden kann. Hierbei wird z. B. ein erstes Implantat als weiteres Gerät und damit als Ultraschallsender und/oder -empfänger und/oder zur Datenverarbeitung und/oder - speicherung verwendet und weist hierfür einen kleinen Energiespeicher, beispielsweise eine kleine Batterie, auf. Das zweite Implantat hat den erfindungsgemäßen, unten im Detail beschriebenen Aufbau und kommuniziert passiv. Das erfindungsgemäße Implantat kann dabei insbesondere dort eingesetzt werden, wo nur sehr wenig Raum zum Einbringen des Implantats zur Verfügung steht, beispielsweise an einer bestimmten Messstelle für einen Körperparameter. Das erfindungsgemäße Implantat stellt somit einen Knoten in einem Netzwerk von miteinander kommunizierenden Implantaten oder von externen Geräten und Implantaten dar.

Nach der vorliegenden Erfindung besonders hervorzuheben ist, dass das Piezoelement gleichzeitig bei der ersten Resonanzfrequenz und der zweiten Resonanzfrequenz anregbar ist. Hierdurch ist die erfindungsgemäße Vorrichtung auch hinsichtlich ihrer Schnelligkeit vorteilhaft.

In einem bevorzugten Ausführungsbeispiel ist das Piezoelement als dünne, rechteckförmige piezoelektrische Schicht, vorzugsweise mit einer Höhe H im Bereich von 100 µm bis 1000 µm, vorzugsweise im Bereich von 250 µm bis 350 µm, ausgebildet, wobei die Länge L, B der Seitenkanten die jeweilige erste und zweite Resonanzfrequenz bestimmen. Hierbei verläuft die Richtung, in die die Höhe der piezoelektrischen Schicht gemessen wird, senkrecht zu den Seitenkanten L, B. Die erforderlichen/gewünschten Resonanzfrequenzen sind durch die Längen L, B der Seitenkanten einfach einstellbar. Die piezoelektrische Schicht kann vorzugsweise Blei-Zirkonat-Titanat (PZT) enthalten.

In einer Weiterbildung der Erfindung weist das Piezoelement insgesamt mindestens zwei jeweils schichtartig ausgebildete Elektroden auf, wobei eine erste Elektrode auf der Unterseite der rechteckförmigen piezoelektrischen Schicht und mindestens eine zweite Elektrode auf der Oberseite der rechteckförmigen piezoelektrischen Schicht angeordnet sind. Hierbei ist die Oberseite der piezoelektrischen Schicht eine Seitenfläche mit der größten Ausdehnung der piezoelektrischen Schicht und die Unterseite die der Oberseite gegenüberliegende Seite der piezoelektrischen Schicht.

In einer bevorzugten Weiterbildung der Erfindung weist das Piezoelement insgesamt mindestens drei jeweils schichtartig ausgebildete Elektroden auf, wobei eine erste Elektrode auf der Unterseite der rechteckförmigen piezoelektrischen Schicht und eine zweite Elektrode und mindestens eine dritte Elektrode, welche galvanisch von der zweiten Elektrode getrennt ist, auf der Oberseite der rechteckförmigen piezoelektrischen Schicht angeordnet sind.

In einem Ausführungsbeispiel können die zweite Elektrode und die mindestens eine dritte Elektrode auf der Oberseite der piezoelektrischen Schicht nebeneinander, beispielsweise als rechteckförmige Schicht, angeordnet sein. Dieser Aufbau ist besonders einfach realisierbar.

Besonders bevorzugt ist es, wenn die auf der Oberseite angeordneten Elektroden kammartig ausgebildete Stege aufweisen, wobei die Stege der zweiten Elektrode und die Stege der dritten Elektrode ineinander greifen, d. h. je ein Steg der zweiten Elektrode liegt in einer Lücke zwischen zwei Stegen der dritten Elektrode und umgekehrt.

Durch eine Anordnung der zweiten Elektrode und der dritten Elektrode auf der Oberseite der piezoelektrischen Schicht vereinfacht sich der schaltungstechnische Aufwand für den gleichzeitigen Sende- und Empfangsbetrieb bei mehreren Frequenzen. Dies ergibt sich daraus, dass kein galvanischer Kontakt zwischen der zweiten Elektrode und der dritten Elektrode auf der Oberseite der piezoelektrischen Schicht besteht, d. h. die beiden Elektroden sind voneinander elektrisch isoliert. Dadurch muss keine zusätzliche Trennung geschaffen werden.

Es ist von Vorteil, wenn ein Verstärker vorgesehen ist, dessen Eingang zu seiner Energieversorgung über einen Gleichrichter mit dem Piezoelement verbunden ist. Mithilfe der bei der ersten Resonanzfrequenz durch das Piezoelement gewandelten elektrischen Energie erzeugt der Verstärker ein elektrisches Ausgangssignal (eine Wechselspannung), durch das das Piezoelement bei der zweiten Resonanzfrequenz anregbar ist und das entsprechende zweite Ultraschallsignal zum Versenden erzeugt. Beispielsweise kann der Verstärker als Klasse-E-Verstärker ausgeführt sein.

Bei einer Verwendung von lediglich einer einzigen Elektrode auf der Oberseite der piezoelektrischen Schicht und einer einzigen Elektrode auf der Unterseite der piezoelektrischen Schicht würden die Elektroden auf der Ober- und Unterseite des Piezoelements durch den Klasse-E-Verstärker kurzgeschlossen werden, was durch die zusätzliche Verwendung eines Zirkulators oder eines Diplexers behoben werden kann. Durch die vorgeschlagene Strukturierung der Elektroden auf der Oberseite der piezoelektrischen Schicht kann eine solche Trennung und somit der damit verbundene schaltungstechnische Aufwand vermieden werden.

Um eine gute Trennung der Anregung des Piezoelements im Sende- und Empfangsmodus zu erzielen, ist die Länge L, B der Seitenkanten der piezoelektrischen Schicht so gewählt, dass der Frequenzabstand zwischen der ersten Resonanzfrequenz und der zweiten Resonanzfrequenz mindestens 100 kHz, vorzugsweise mindestens 450 kHz, beträgt.

Um eine Übertragung von Daten von dem Implantat zu einem weiteren Gerät, beispielsweise einem externen Empfänger oder einem weiteren Implantat, zu realisieren, ist es von Vorteil, wenn der Verstärker einen Modulator aufweist, durch den das Ausgangssignal zur Übertragung von Daten modulierbar ist. Hierfür können alle gängigen Modulationsverfahren wie Frequenz-, Amplituden- oder Phasenmodulation verwendet werden.

Die obige Aufgabe wird ferner durch ein Verfahren mit den Merkmalen nach Anspruch 11 gelöst. Das erfindungsgemäße Verfahren besitzt die oben bereits im Zusammenhang mit dem Implantat genannten Vorteile. Hierfür wird vorzugsweise mittels des Verstärkers unter Einsatz der durch die ersten Ultraschallsignale erzeugten elektrischen Energie eine Wechselspannung als Ausgangssignal erzeugt, mit der das Piezoelement bei der zweiten Resonanzfrequenz anregbar ist.

In einem besonders bevorzugten Ausführungsbeispiel moduliert der oben beschriebene Modulator das elektrische Ausgangssignal des oben beschriebenen Verstärkers.

Mit einer entsprechenden Modulation können auch mittels des zu dem Implantat ausgesandten ersten Ultraschallsignals Daten zu dem Implantat übertragen werden. Damit ist das beschriebene Implantat in der Lage bidirektional mit einem weiteren Gerät zu kommunizieren. Mit dem beschriebenen Piezoelement ist eine bidirektionale Kommunikation des Implantats sowohl im Wechselbetrieb (halbduplex) als auch im Gegenbetrieb (vollduplex) möglich.

Das erfindungsgemäße Implantat und das erfindungsgemäße Verfahren werden nachfolgend anhand von Beispielen und Figuren erläutert. Dabei bilden alle abgebildeten und/oder beschriebenen Merkmale den Gegenstand der Erfindung, auch unabhängig von ihrer Zusammenfassung in den Ansprüchen oder deren Rückbeziehung.

Es zeigen schematisch:
- Fig. 1: ein erstes Ausführungsbeispiel eines erfindungsgemäßen Implantats sowie eine externe Sende- und Empfangseinheit als Blockschaltbild,
- Fig. 2: das Ausführungsbeispiel eines Implantats gemäß Fig. 1 sowie die dort gezeigte Sende- und Empfangseinheit im Detail als Blockschaltbild,
- Fig. 3: eine piezoelektrische Schicht in einer perspektivischen Ansicht von der Seite,
- Fig. 4: den Impedanzverlauf (in Abhängigkeit von der Frequenz) eines Piezoelements eines erfindungsgemäßen Implantats nach Fig. 1,
- Fig. 5: den Phasenverlauf eines Piezoelements eines erfindungsgemäßen Implantats nach Fig. 1,
- Fig. 6: ein erstes Ausführungsbeispiel eines Piezoelements für ein erfindungsgemäßes Implantat in einer Ansicht von unten,
- Fig. 7: das Piezoelement gemäß Fig. 6 in einer Ansicht von oben,
- Fig. 8: das Piezoelement gemäß Fig. 6 in einer Ansicht von der Seite,
- Fig. 9: ein erstes Ausführungsbeispiel eines erfindungsgemäßen Implantats mit einem in den Fig. 6 bis 8 dargestellten Piezoelement in einem Blockschaltbild
- Fig. 10: ein zweites Ausführungsbeispiel eines Piezoelements für ein erfindungsgemäßes Implantat in einer Ansicht von oben,
- Fig. 11: das Piezoelement gemäß Fig. 10 in einer Ansicht von unten,
- Fig. 12: das Piezoelement gemäß Fig. 10 in einer Ansicht von der Seite,
- Fig. 13: ein zweites Ausführungsbeispiel eines erfindungsgemäßen Implantats mit einem in den Fig. 10 bis 12 dargestellten Piezoelement in einem Blockschaltbild,
- Fig. 14: ein drittes Ausführungsbeispiel eines Piezoelements für ein erfindungsgemäßes Implantat in einer Ansicht von oben,
- Fig. 15: das Piezoelement gemäß Fig. 14 in einer Ansicht von unten,
- Fig. 16: das Piezoelement gemäß Fig. 14 in einer Ansicht von der (kürzeren) Seite und
- Fig. 17: ein drittes Ausführungsbeispiel eines erfindungsgemäßen Implantats mit einem in den Fig. 14 bis 16 dargestellten Piezoelement in einem Blockschaltbild.

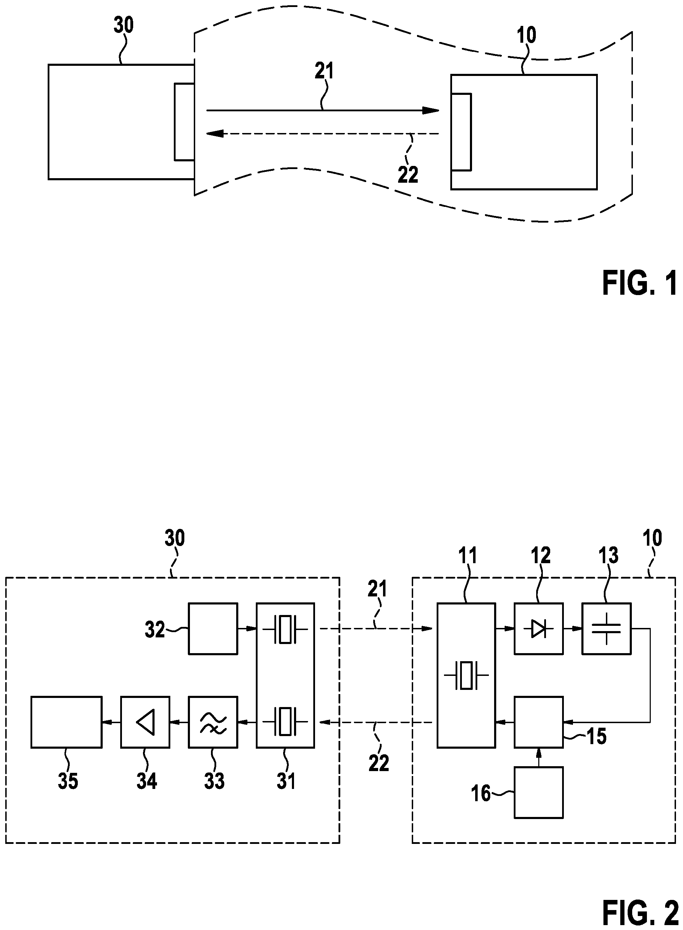

Ein System aus einem ersten Ausführungsbeispiel eines erfindungsgemäßen Implantats 10 und einer Sende- und Empfangseinheit 30 eines weiteren Geräts (nicht abgebildet) ist in den Figuren 1 und 2 dargestellt. Bei dem weiteren Gerät kann es sich um ein Programmiergerät, ein Patientengerät oder ein weiteres Implantat handeln. Von der Sende- und Empfangseinheit 30 des weiteren Geräts werden erste Ultraschallsignale 21 versendet, welche durch den Körper das erfindungsgemäße Implantat 10 erreichen und dort mittels des Piezoelements 11 empfangen werden. Das gleiche Piezoelement 11 dient auch, wie nachfolgend genauer beschrieben werden soll, als Sender für zweite Ultraschallsignale 22 von dem Implantat 10 zu der Sende- und Empfangseinheit 30 des weiteren Geräts zur Übertragung von Daten von dem Implantat 10 zu dem weiteren Gerät.

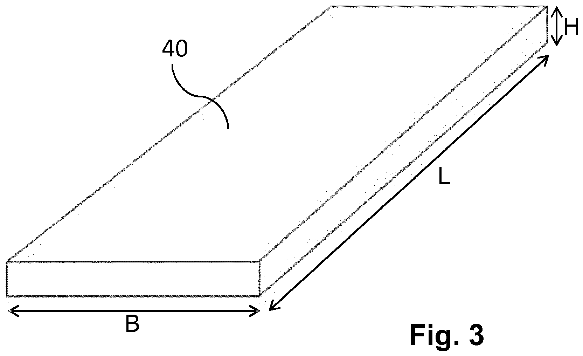

Das Piezoelement 11 des erfindungsgemäßen Implantats umfasst eine in Fig. 3 dargestellte, plattenförmige, rechteckige, keramische piezoelektrische Schicht 40 mit einer bestimmten, vorgegebenen Länge L, einer bestimmten, vorgegebenen Breite B und einer bestimmten, vorgegebenen Höhe H. Die Höhe H der piezoelektrischen Schicht ist klein gegenüber der Länge L und der Breite B. Die Höhe H beträgt beispielsweise 300 µm. Die piezoelektrische Schicht 40 wird demnach auch als dünn bezeichnet.

Die Resonanzfrequenzen des Piezoelements 11 werden durch die Länge L und die Breite B der piezoelektrischen Schicht 40 vorgegeben. Durch Anregung der piezoelektrischen Schicht 40 bei den Serienresonanzfrequenzen kann besonders effizient mechanische in elektrische Energie und umgekehrt gewandelt werden.

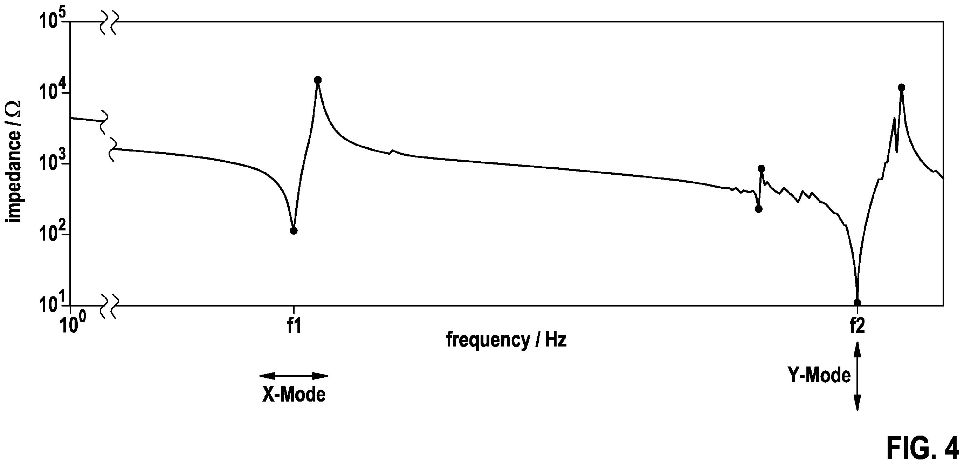

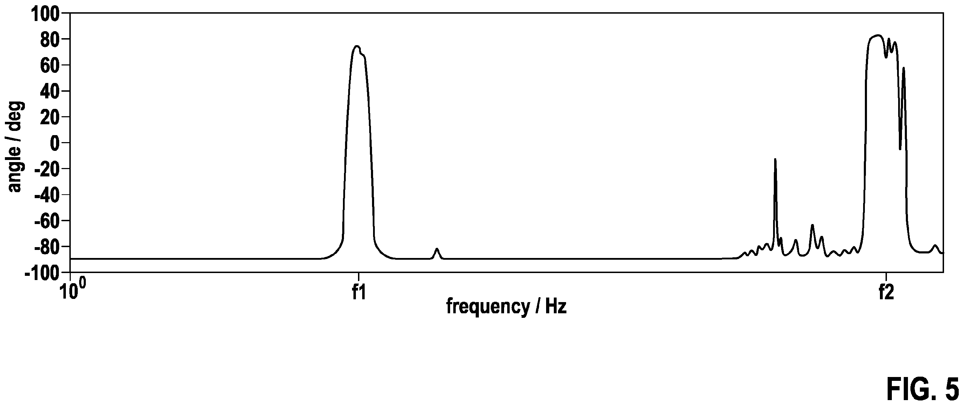

Beispielsweise weist eine piezoelektrische Schicht 40 mit einer Länge L von 5 mm, einer Breite B von 2 mm und einer Höhe H von 300 µm aus dem Material Blei-Zirkonat-Titanat (PZT) eine Serienresonanzfrequenz von f1 = 320 kHz (X-Mode) und eine Serienresonanzfrequenz von f2 = 800 kHz (Y-Mode) auf. Der Verlauf des Absolutbetrags der Impedanz in Abhängigkeit von der Frequenz einer derartigen piezoelektrischen Schicht ist in Fig. 4 dargestellt. Entsprechend zeigt Fig. 5 den Verlauf der Phasenabhängigkeit zwischen Strom und Spannung von der Frequenz für dieselbe Schicht. Zur Messung dieser Verläufe wird die piezoelektrische Schicht 40 an ihrer Ober- und Unterseite kontaktiert, so dass die elektrischen Feldlinien von der Ober- zur Unterseite bzw. von der Unter- zur Oberseite (entsprechend der Polarität der angelegten Spannung) der Schicht 40 verlaufen, und mit Hilfe eines Impedanzanalysators untersucht. Die Sprünge in einem Impedanzverlauf weisen dabei jeweils auf eine Resonanz bei der entsprechenden Frequenz hin. Ein lokales Minimum kennzeichnet dabei jeweils eine Serienresonanzfrequenz der Keramik. Das darauf folgende lokale Maximum kennzeichnet die dazu gehörige Parallelresonanzfrequenz.

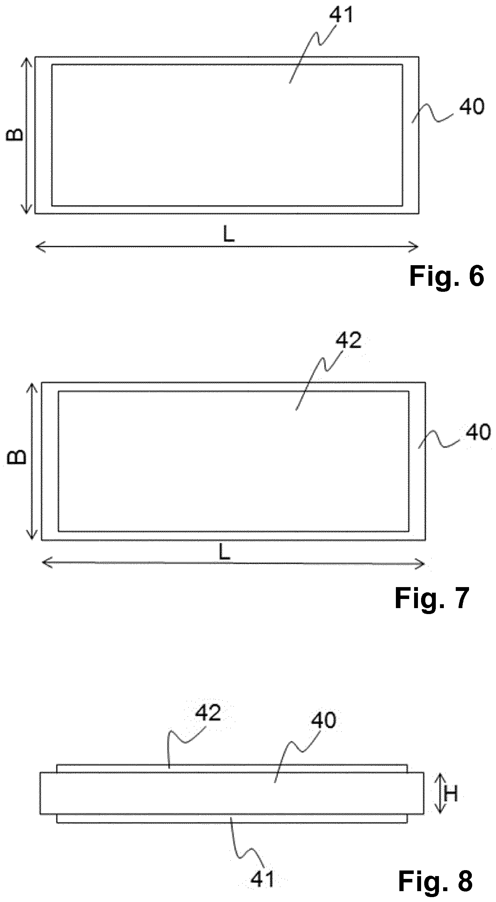

Die piezoelektrische Schicht 40 des Piezoelements 11 weist, wie in den Figuren 6 bis 8 dargestellt, in einem ersten Ausführungsbeispiel zwei schichtartig ausgeführte Elektroden auf, nämlich auf der Unterseite eine erste, schichtartig ausgebildete Elektrode 41, wie in Fig. 6 gezeigt, und auf der Oberseite eine zweite, schichtartig ausgebildete Elektrode 42 (vgl. Fig. 7). Hierbei sind die Oberseite und die Unterseite der piezoelektrischen Schicht 40 gegenüberliegende Seitenflächen mit der größten Ausdehnung. Die Elektroden 42, 41 nehmen fast die gesamte Oberfläche der Oberseite bzw. der Unterseite der piezoelektrischen Schicht 40 ein.

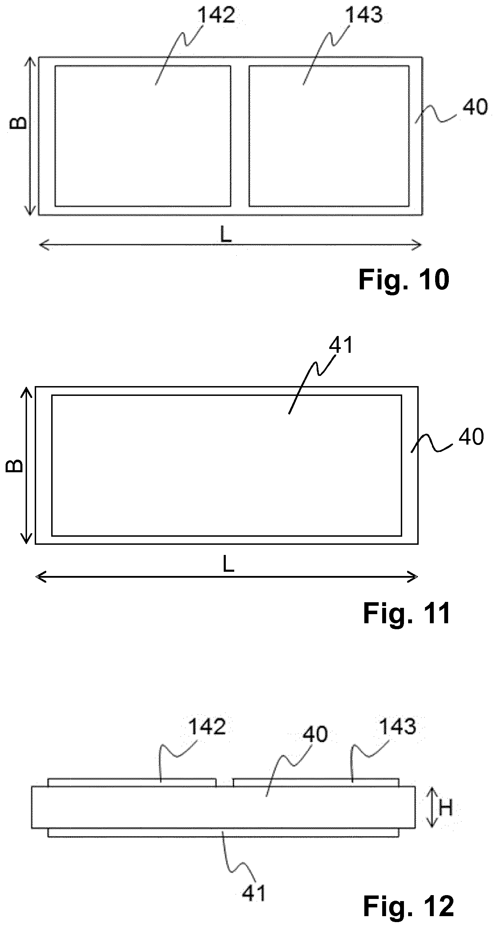

Demgegenüber ist bei dem in den Figuren 10 bis 12 dargestellten zweiten Ausführungsbeispiel eines Piezoelements 11 auf der Oberseite der piezoelektrischen Schicht 40, wie in Fig. 10 gezeigt, eine zweite Elektrode 142 und eine dritte Elektrode 143 in der Form von zwei nebeneinanderliegend angeordneten Schichten aufgebracht. Auf der Unterseite ist analog zu dem ersten Ausführungsbeispiel eines Piezoelements 11, wie in Fig. 11 gezeigt, lediglich eine einzige erste Elektrode 41 vorgesehen. Die zweite Elektrode 142 und die dritte Elektrode 143 sind dabei galvanisch voneinander getrennt.

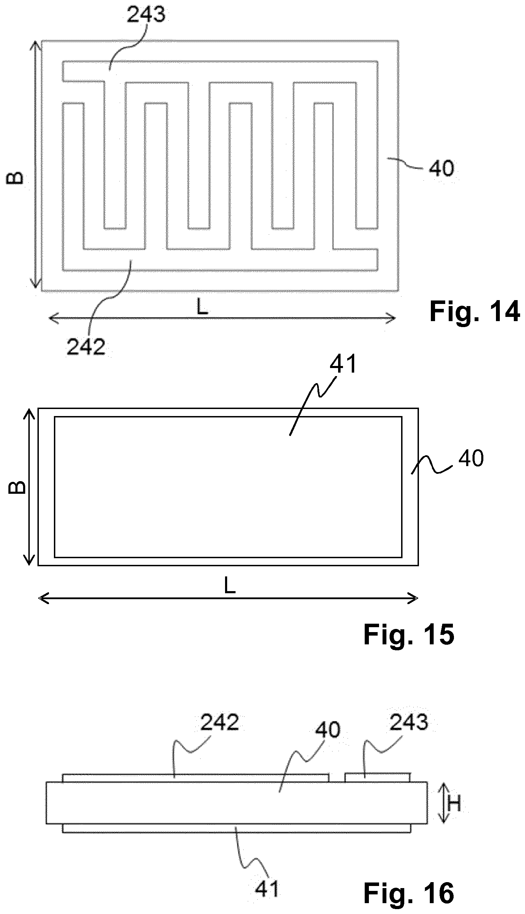

Das in den Figuren 14 bis 16 dargestellte dritte Ausführungsbeispiel eines Piezoelements 11 unterscheidet sich von dem zweiten Ausführungsbeispiel, wie in Fig. 14 gezeigt, in der Struktur der auf der Oberseite der piezoelektrischen Schicht 40 angeordneten zweiten Elektrode 242 und dritten Elektrode 243. Diese Elektroden 242, 243 weisen kammartig angeordnete Stege auf, wobei jeweils ein Steg der zweiten Elektrode 242 in einer Lücke zwischen zwei Stegen der dritten Elektrode 243 und umgekehrt angeordnet ist. Auf der Unterseite der piezoelektrischen Schicht 40 ist, wie in Fig. 15 gezeigt, analog zum ersten und zweiten Ausführungsbeispiel lediglich eine einzige Elektrode 41 vorgesehen. Die zweite Elektrode 242 und die dritte Elektrode 243 sind dabei galvanisch voneinander getrennt.

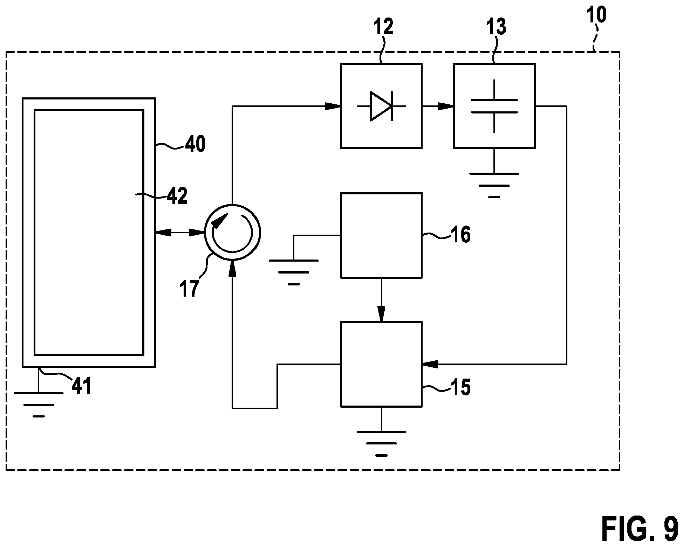

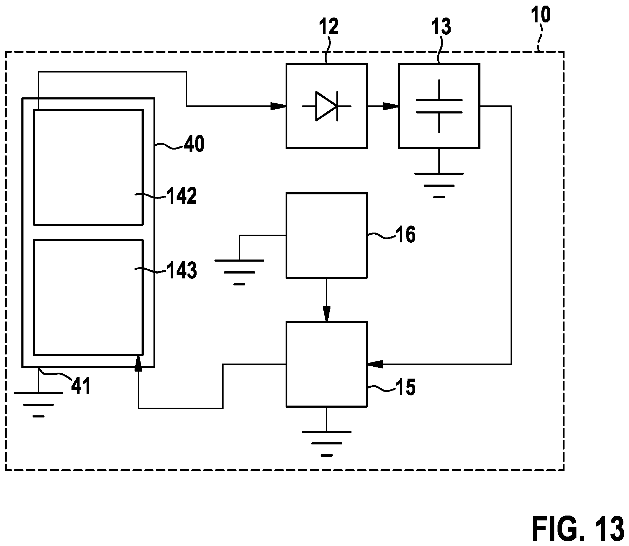

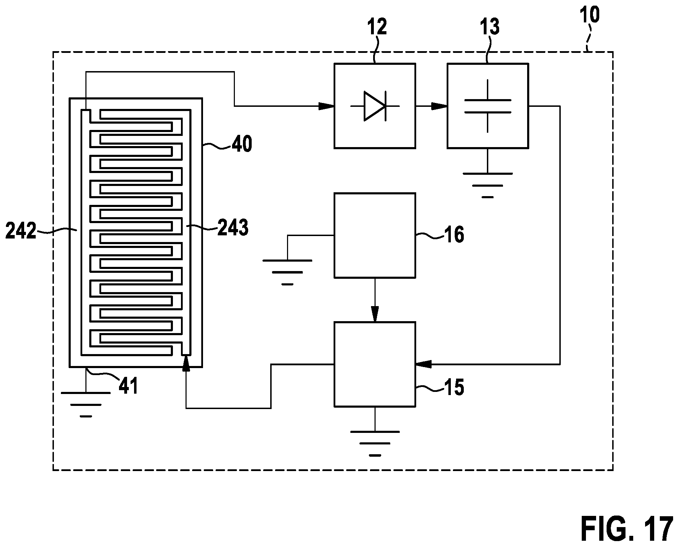

Die Verschaltung der Elektroden des Piezoelements 11 ist in den Figuren 9, 13 und 17 jeweils anhand eines Blockschaltbildes erläutert. Generell gibt es zwei unterschiedliche Schaltungsvarianten: eine erste Schaltungsvariante für ein Piezoelement 11 mit zwei Elektroden wie in Fig. 9 gezeigt und eine zweite Schaltungsvariante für ein Piezoelement 11 mit drei Elektroden wie in den Figuren 13 und 17 dargestellt.

Die Fig. 9 zeigt die erste Schaltungsvariante. Während die erste Elektrode 41 auf Masse liegt, ist die zweite Elektrode 42 mit einem Zirkulator 17 verbunden, der die Frequenz f1 des eingehenden Signals von der Frequenz f2 des ausgehenden Signals trennt. Das Piezoelement empfängt ein erstes Ultraschallsignal 21 mit einer Frequenz f1, das in ein entsprechendes eingehendes elektrisches Signal mit einer Frequenz f1 umgewandelt wird. Der Zirkulator 17 leitet das eingehende Signal mit der Frequenz f1 an einen Gleichrichter 12, beispielsweise eine Diode, weiter und führt dieses anschließend einem Kondensator 13, der als Energiezwischenspeicher dient, zu. An dem Kondensator 13 kann das eingehende Signal abgegriffen werden. Über den anderen Anschluss des Zirkulators 17 wird ein vom Frequenzerzeuger 16 erzeugtes und durch den Klasse-E-Verstärker 15 verstärktes ausgehendes Signal mit der Frequenz f2 an die zweite Elektrode 42 weitergeleitet. Die Verbindung zwischen dem Kondensator 13 und dem Klasse-E-Verstärker 15 dient der Energieversorgung des Verstärkers. Das Piezoelement 11 wandelt das ausgehende elektrische Signal in das zweite Ultraschallsignal 22 um. Zudem ist ein nicht dargestellter Modulator vorgesehen, welcher das ausgehende Signal mittels einer Frequenz-, Amplituden- oder Phasenmodulation moduliert, um Daten von dem Implantat 10 zu dem weiteren Gerät zu übertragen.

Weiterhin zeigt Fig. 13 die Verschaltung der Elektroden 41, 142 und 143 und Fig. 17 die Verschaltung der Elektroden 41, 242 und 243 nach der zweiten Schaltungsvariante. Auch hier liegt in beiden Fällen die erste Elektrode 41 auf Masse. Die zweite Elektrode 142, 242 ist mit dem Gleichrichter 12 verbunden, der ein aus dem ersten Ultraschallsignal 21 mit der Frequenz f1 erzeugtes eingehendes elektrisches Signal gleichrichtet. Anschließend wird das gleichgerichtete Signal einem Kondensator 13, der als Energiespeicher dient, zugeführt. An dem Kondensator 13 kann das eingehende Signal in Form der Frequenz f1 abgegriffen werden. Die dritte Elektrode 143, 243 ist jeweils mit dem Klasse-E-Verstärker 15 verbunden und ist zum Erzeugen des zweiten Ultraschallsignals 22 und Senden des zweiten Ultraschallsignals 22 mit der Frequenz f2 vorgesehen, welches elektrisch durch den jeweils mit der dritten Elektrode 143, 243 verbundenen Frequenzerzeuger 16 bereitgestellt und durch den Klasse-E-Verstärker 15 verstärkt wurde. Die Verbindung zwischen dem Kondensator 13 und dem Klasse-E-Verstärker 15 dient der Energieversorgung des Verstärkers. Auch bei dieser Schaltungsvariante ist ein nicht dargestellter Modulator vorgesehen, welcher das ausgehende Signal mittels einer Frequenz-, Amplituden- oder Phasenmodulation moduliert, um Daten von dem Implantat 10 zu dem weiteren Gerät zu übertragen.

Die Sende- und Empfangseinheit 30 des weiteren Geräts , beispielsweise ein außerhalb des Körpers befindliches Patientengerät, sendet mittels eines Sende-/Empfangsschallwandlers 31 ein erstes Ultraschallsignal 21, beispielsweise bei einer Frequenz f1 von 320 kHz. Dieses erste Ultraschallsignal entspricht der Serienresonanzfrequenz des Piezoelements 11 des Implantats 10 und wird daher besonders effizient von dem Piezoelement 11 empfangen und in eine entsprechende elektrische Wechselspannung gewandelt. Diese elektrische Wechselspannung wird mittels eines Gleichrichters 12, der mit dem Piezoelement 11 verbunden ist, gleich gerichtet und in einem mit dem Gleichrichter 12 verbundenen Kondensator 13 zwischengespeichert. Die in dem Kondensator 13 gespeicherte elektrische Energie in Form einer Gleichspannung wird nun mittels eines Klasse-E-Verstärkers 15 und einem Oszillator 16 in eine Wechselspannung mit einer Frequenz von 800 kHz gewandelt. Die zur Übertragung anstehenden Daten werden mittels eines bekannten Modulationsverfahrens mit einem in den Klasse-E-Verstärker 15 integrierten Modulator aufmoduliert und über das Piezoelement 11 bei der zweiten Serienresonanzfrequenz f2 in ein zweites Ultraschallsignal 22 gewandelt. Diese Wandlung der elektrischen Energie in mechanische Energie erfolgt durch das Piezoelement 11 besonders effizient, da es bei der zweiten Serienresonanzfrequenz f2 betrieben wird. In der Sende- und Empfangseinheit 30 des weiteren Geräts wird das zweite Ultraschallsignal 22 mittels Sende-/Empfangsschallwandler 31 empfangen, gleichgerichtet und demoduliert. Hierfür sind ein Hochpassfilter 33, Verstärker 34 und ein damit verbundener Demodulator 35 vorgesehen. Hierdurch werden die übertragenen Daten aus dem zweiten Ultraschallsignal 22 extrahiert. Weiter kann ein nicht dargestellter Speicher für die mit dem zweiten Ultraschallsignal 22 übertragenen Daten sowie eine nicht dargestellte Recheneinheit (Mikrocontroller, µC) zur Verarbeitung dieser Daten vorgesehen sein.

Die Sende- und Empfangseinheit 30 des weiteren Geräts ist im Wesentlichen analog zum Implantat aufgebaut. Es besitzt jedoch vorzugsweise mindestens zwei in einer Schallwandlereinheit 31 vorgesehene Schallwandler, einen ersten Schallwandler zum Erzeugen und Senden des ersten Ultraschallsignals 21 und einen zweiten Schallwandler zum Empfangen und Umwandeln des zweiten Ultraschallsignals 22. Ein derartiger Aufbau der Sende- und Empfangseinheit 30 des weiteren Geräts ist deshalb von Vorteil, weil durch die Trennung der Vorgänge Senden des ersten Ultraschallsignals 21 und Empfangen des zweiten Ultraschallsignals 22 durch mindestens zwei verschiedene Schallwandler die Komplexität der Signalverarbeitung reduziert wird.

In einer alternativen Ausführung kann die Sende- und Empfangseinheit 30 des weiteren Geräts, analog zu dem Implantat 10, auch mit einem kombinierten Schallwandler gemäß der Figuren 6 bis 8, 10 bis 12 und 14 bis 16 aufgebaut werden. Diese Variante kann vorteilhaft sein, wenn es sich bei dem weiteren Gerät ebenfalls um ein Implantat handelt.

Das erfindungsgemäße Implantat 10 kann raumsparend konstruiert werden, da lediglich aufgrund der mit dem Ultraschallsignal übertragenen Energie insbesondere bei Anwendungen mit umfangreicher Kommunikation die Spannungsquelle kleinbauender realisiert werden kann. Zudem haben die zur Datenübertragung verwendeten Ultraschallsignale eine hohe Reichweite auch im Körper einer Person. Die Elemente des erfindungsgemäßen Implantats können in ein metallisches Gehäuse, z. B. aus Titan, integriert werden.

## Patentansprüche

1. Implantat (10) mit einem Empfänger für von einer Sendeeinheit (30) eines weiteren Geräts ausgesandte erste Ultraschallsignale (21) in Form eines Piezoelements (11), welches durch die ersten Ultraschallsignale (21) bei einer ersten Resonanzfrequenz (f1) angeregt wird und hierbei die mit den ersten Ultraschallsignalen (21) übertragene mechanische Energie in elektrische Energie wandelt, wobei das Piezoelement (11) zusätzlich bei einer zweiten Resonanzfrequenz (f2) anregbar ist und bei der zweiten Resonanzfrequenz (f2) als Sender von zweiten Ultraschallsignalen (22) betreibbar ist, **dadurch gekennzeichnet, dass** die erste Resonanzfrequenz (f1) eine erste Serienresonanzfrequenz und dass die zweite Resonanzfrequenz (f2) eine zweite Serienresonanzfrequenz ist, welche sich von der ersten Serienresonanzfrequenz unterscheidet.

2. Implantat nach Anspruch 1, **dadurch gekennzeichnet, dass** das Piezoelement (11) gleichzeitig bei der ersten Resonanzfrequenz (f1) und der zweiten Resonanzfrequenz (f2) anregbar ist.

3. Implantat nach einem der vorhergehenden Ansprüche, **dadurch gekennzeichnet, dass** das Piezoelement (11) als dünne, rechteckförmige piezoelektrische Schicht (40), vorzugsweise mit einer Höhe (H) im Bereich von 100 µm bis 1000 µm ausgebildet ist, wobei die Länge der Seitenkanten (L, B) die jeweilige erste und zweite Resonanzfrequenz (f1, f2) bestimmen.

4. Implantat nach Anspruch 3, **dadurch gekennzeichnet, dass** auf der Unterseite einer piezoelektrischen Schicht (40) des Piezoelements (11) eine erste Elektrode (41) und auf der Oberseite der piezoelektrischen Schicht (40) mindestens eine zweite Elektrode (42, 142, 242) angeordnet sind.

5. Implantat nach Anspruch 3, **dadurch gekennzeichnet, dass** auf der Unterseite einer piezoelektrischen Schicht (40) des Piezoelements (11) eine erste Elektrode (41) und auf der Oberseite der piezoelektrischen Schicht (40) eine zweite Elektrode (142, 242) und mindestens eine galvanisch von der zweiten Elektrode getrennte dritte Elektrode (143, 243) angeordnet sind.

6. Implantat nach Anspruch 5, **dadurch gekennzeichnet, dass** die zweite Elektrode (142) und die mindestens eine dritte Elektrode (143) auf der Oberseite der piezoelektrischen Schicht (40) nebeneinander als rechteckförmige Schicht angeordnet sind.

7. Implantat nach Anspruch 5, **dadurch gekennzeichnet, dass** die zweite Elektrode (242) und die dritte Elektrode (243) auf der Oberseite der piezoelektrischen Schicht (40) kammartig ausgebildete Stege aufweisen, wobei die Stege der zweiten Elektrode (242) und der dritten Elektrode (243) ineinander greifen.

8. Implantat nach einem der vorhergehenden Ansprüche, **dadurch gekennzeichnet, dass** das Piezoelement (11) mit einem Verstärker (15) verbunden ist, der mit der bei der ersten Resonanzfrequenz (f1) gewandelten elektrischen Energie ein elektrisches Ausgangssignal erzeugt, durch das das Piezoelement (11) bei der zweiten Resonanzfrequenz (f2) anregbar ist und das entsprechende zweite Ultraschallsignal (22) zum Versenden erzeugt.

9. Implantat nach einem der vorhergehenden Ansprüche, **dadurch gekennzeichnet, dass** der Frequenzabstand zwischen der ersten Resonanzfrequenz (f1) und der zweiten Resonanzfrequenz (f2) mindestens 100 kHz beträgt.

10. Implantat nach einem der vorhergehenden Ansprüche, **dadurch gekennzeichnet, dass** der Verstärker (15) einen Modulator aufweist, durch den das Ausgangssignal zur Übertragung von Daten modulierbar ist.

11. Verfahren zum Betreiben eines Implantats nach den Ansprüchen 1 bis 10, wobei das Piezoelement (11) bei der ersten Serienresonanzfrequenz (f1) angeregt wird und die von der Sendeeinheit (30) des weiteren Geräts ausgesandten ersten Ultraschallsignale (21) empfängt sowie mittels der erzeugten elektrischen Energie bei der zweiten Serienresonanzfrequenz (f2) angeregt wird und hierdurch die zweiten Ultraschallsignale (22) aussendet.

12. Verfahren nach Anspruch 11, **dadurch gekennzeichnet, dass** der Modulator das elektrische Ausgangssignal des Verstärkers (15) moduliert, um eine Datenübertragung zu realisieren.

## Claims

1. An implant (10) comprising a receiver for receiving first ultrasound signals (21) emitted by a transmitting unit (30) of a further apparatus, which receiver is in the form of a piezo element (11) which is excited by the first ultrasound signals (21) at a first resonance frequency (f1) and in so doing converts mechanical energy transferred with the first ultrasound signals (21) into electrical energy, the piezo element (11) additionally being excitable at a second resonance frequency (f2) and being operable at the second resonance frequency (f2) as a transmitter of second ultrasound signals (22), **characterised in that** the first resonance frequency (f1) is a first series resonance frequency, and **in that** the second resonance frequency (f2) is a second series resonance frequency, which differs from the first series resonance frequency.

2. The implant according to claim 1, **characterised in that** the piezo element (11) is excitable at the first resonance frequency (f1) and the second resonance frequency (f2) simultaneously.

3. The implant according to one of the preceding claims, **characterised in that** the piezo element (11) is designed as a thin rectangular piezoelectric layer (40), preferably with a height (H) ranging from 100 µm to 1000 µm, the lengths of the side edges (L, B) determining the first and second resonance frequencies (f1, f2).

4. The implant according to claim 3, **characterised in that** a first electrode (41) is arranged on the underside of a piezoelectric layer (40) of the piezo element (11) and at least one second electrode (42, 142, 242) is arranged on the upper side of the piezoelectric layer (40).

5. The implant according to claim 3, **characterised in that** a first electrode (41) is arranged on the underside of a piezoelectric layer (40) of the piezo element (11) and a second electrode (142, 242) and at least one third electrode (143, 243) galvanically separated from the second electrode are arranged on the upper side of the piezoelectric layer (40).

6. The implant according to claim 5, **characterised in that** the second electrode (142) and the at least one third electrode (143) are arranged adjacently in the form of a rectangular layer on the upper side of the piezoelectric layer (40).

7. The implant according to claim 5, **characterised in that** the second electrode (242) and the third electrode (243) on the upper side of the piezoelectric layer (40) comprise comb-like ribs, the comb-like ribs of the second electrode (242) and of the third electrode (243) engaging with one another.

8. The implant according to one of the preceding claims, **characterised in that** the piezo element (11) is connected to an amplifier (15), which generates an electrical output signal with the electrical energy that is converted at the first resonance frequency (f1), by means of which electrical output signal the piezo element (11) is excitable at the second resonance frequency (f2) and generates the corresponding second ultrasound signal (22) to be transmitted.

9. The implant according to one of the preceding claims, **characterised in that** the frequency distance between the first resonance frequency (f1) and the second resonance frequency (f2) is at least 100 kHz.

10. The implant according to one of the preceding claims, **characterised in that** the amplifier (15) comprises a modulator, by means of which the output signal can be modulated to cause a transfer of data.

11. A method for operating an implant according to claims 1 to 10, wherein the piezo element (11) is excited at the first series resonance frequency (f1) and receives the first ultrasound signals (21) emitted by the transmitting unit (30) of the further apparatus and is excited at the second series resonance frequency (f2) by means of the generated electrical energy and hereby emits the second ultrasound signals (22).

12. The method according to claim 11, **characterised in that** the modulator modulates the electrical output signal of the amplifier (15) in order to cause a transfer of data.

## Revendications

1. Implant (10) doté d'un récepteur pour des premiers signaux ultrasonores (21) émis par une unité émettrice (30) d'un autre appareil sous forme d'un élément piézo (11), lequel est excité par les premiers signaux ultrasonores (21) à une première fréquence de résonance (f1) et convertit ainsi l'énergie mécanique transmise avec les premiers signaux ultrasonores (21) en énergie électrique, où l'élément piézo (11) peut être excité en outre à une deuxième fréquence de résonance (f2) et peut être activée en tant qu'émetteur de deuxième signaux ultrasonores (22) à la deuxième fréquence de résonance (f2), **caractérisé en ce que** la première fréquence de résonance (f1) est une première fréquence de résonance série et que la deuxième fréquence de résonance (f2) est une deuxième résonance série, laquelle est différente de la première résonance série.

2. Implant selon la revendication 1, **caractérisé en ce que** l'élément piézo (11) peut être excité simultanément à la première fréquence de résonance (f1) et à la deuxième fréquence de résonance (f2).

3. Implant selon l'une des revendications précédentes, **caractérisé en ce que** l'élément piézo (11) est conçu sous forme d'une couche piézoélectrique (40) fine, en forme de rectangle, de préférence, avec une hauteur (H) dans la plage de 100 µm à 1000 µm, où les longueurs des côtés latéraux (L, B) déterminent respectivement la première et la deuxième fréquence de résonance (f1, f2).

4. Implant selon la revendication 3, **caractérisé en ce qu'**une première électrode (41) est disposée sur la face inférieure d'une couche piézoélectrique (40) de l'élément piézo (11) et au moins une deuxième électrode (42, 142, 242) est disposée sur la face supérieure de la couche piézoélectrique (40).

5. Implant selon la revendication 3, **caractérisé en ce qu'**une première électrode (41) est disposée sur la face inférieure d'une couche piézoélectrique (40) de l'élément piézo (11) et au moins une deuxième électrode (42, 142, 242) et au moins une troisième électrode (143, 243), séparée de manière galvanique de la deuxième électrode, sont disposées sur la face supérieure de la couche piézoélectrique (40).

6. Implant selon la revendication 5, **caractérisé en ce que** la deuxième électrode (142) et l'au moins une troisième électrode (143) sont disposées sur la face supérieure de la couche piézoélectrique (40) l'une à côté de l'autre, sous forme d'une couche de forme rectangulaire

7. Implant selon la revendication 5, **caractérisé en ce que** la deuxième électrode (242) et l'au moins une troisième électrode (243) présentent des tiges en forme de peigne sur la face supérieure de la couche piézoélectrique (40), où les tiges de la deuxième électrode (242) et de la troisième électrode (243) s'engagent les unes dans les autres.

8. Implant selon l'une des revendications précédentes, **caractérisé en ce que** l'élément piézo (11) est relié à un amplificateur (15) qui génère un signal de sortie électrique avec l'énergie électrique transformée à la première fréquence de résonance (f1), par lequel l'élément piézo (11) peut être excité à la deuxième fréquence de résonance (f2) et génère le deuxième signal ultrasonore (22) correspondant pour l'envoi.

9. Implant selon l'une des revendications précédentes, **caractérisé en ce que** l'écart de fréquence entre la première fréquence de résonance (f1) et la deuxième fréquence de résonance (f2) est d'au moins 100 kHz.

10. Implant selon l'une des revendications précédentes, **caractérisé en ce que** l'amplificateur (15) présente un modulateur par lequel le signal de sortie peut être modulé pour la transmission de données.

11. Procédé de mise en œuvre d'un implant selon les revendications 1 à 10, dans lequel l'élément piézo (11) est excité à la première fréquence de résonance série (f1) et réceptionne les premiers signaux ultrasonores (21) envoyés par l'unité émettrice (30) de l'autre appareil, et excite également la deuxième fréquence de résonance série (12) au moyen de l'énergie électrique générée et de ce fait émet les deuxièmes signaux ultrasonores (22).

12. Procédé selon la revendication 11, **caractérisé en ce que** le modulateur module le signal de sortie électrique de l'amplificateur (15) afin de réaliser une transmission de données.
